# EUROPEAN PATENT SPECIFICATION

(11) **EP 1 753 437 B1**
(45) Date of publication and mention of the grant of the patent: **25.12.2013**
(21) Application number: 05754137.7
(22) Date of filing: 18.05.2005
(51) Int. Cl.: A61M 1/28, A61K 9/50, A61K 33/00

(54) **BICARBONATE-BASED PERITONEAL DIALYSIS SOLUTIONS**
PERITONEALDIALYSELÖSUNGEN AUF BICARBONATBASIS
SOLUTIONS A BASE DE BICARBONATE POUR DIALYSE PERITONEALE

(30) Priority: 10.06.2004 US 866206
(43) Date of publication of application: 21.02.2007
(73) Proprietor: Baxter International Inc., Deerfield, IL 60015 (US); Baxter Healthcare S.A., 8152 Glattpark (Opfikon) (CH)
(72) Inventor: DEGREVE, Bart, B-6040 Jumet (BE); GERICKE, Marion, B-1745 Opwijk (BE); FAICT, Dirk, B-9968 Assenede (BE); DUPONCHELLE, Annick, B-1150 Brussels (BE)
(74) Representative: Potter Clarkson LLP
(86) International application number: PCT/US2005/017995
(87) International publication number: WO 2006/001962

(56) References cited:
- WO-A-01/89478
- US-A1- 2002 012 707
- US-B1- 6 689 393

## Description

### BACKGROUND OF THE INVENTION

The present invention relates generally to solutions used for dialysis therapy.

Due to disease, insult or other causes, a person's renal system can fail. In renal failure of any cause, there are several physiological derangements. The balance of water, minerals and the excretion of daily metabolic load are no longer possible in renal failure. During renal failure, toxic end products of nitrogen metabolism (e.g., urea, creatinine, uric acid, and others) can accumulate in blood and tissues.

Kidney failure and reduced kidney function have been treated with dialysis. Dialysis removes waste, toxins and excess water from the body that would otherwise have been removed by normal functioning kidneys. Dialysis treatment for replacement of kidney functions is critical to many people because the treatment is life saving. One who has failed kidneys could not continue to live without replacing at least the filtration functions of the kidneys.

Hemodialysis, hemofiltration and peritoneal dialysis are three types of dialysis therapies generally used to treat loss of kidney function. Hemodialysis treatment removes waste, toxins and excess water directly from the patient's blood. The patient is connected to a hemodialysis machine and the patient's blood is pumped through the machine. For example, needles or catheters can be inserted into the patient's veins and arteries to connect the blood flow to and from the hemodialysis machine. As blood passes through a dialyzer in the hemodialysis machine, the dialyzer removes the waste, toxins and excess water from the patient's blood and returns the blood to infuse back into the patient. A large amount of dialysate, for example about 90-120 liters, is used by most hemodialysis machines to dialyze the blood during a single hemodialysis therapy. The spent dialysate is then discarded. Hemodialysis treatment lasts several hours and is generally performed in a treatment center about three times per week.

Hemofiltration is a convection-based blood cleansing technique. Blood access can be venovenous or arteriovenous. As blood flows through the hemofilter, a transmembrane pressure gradient between the blood compartment and the ultrafiltrate compartment causes plasma water to be filtered across the highly permeable membrane. As the water crosses the membrane, it convects small and large molecules across the membrane and thus cleanses the blood. An excessive amount of plasma water is eliminated by filtration. Therefore, in order to keep the body water balanced, fluid must be substituted continuously by a balanced electrolyte solution (replacement or substitution fluid) infused intravenously. This substitution fluid can be infused either into the arterial blood line leading to the hemofilter (predilution) or into the venous blood line leaving the hemofilter.

Peritoneal dialysis utilizes a sterile dialysis solution or "dialysate", which is infused into a patient's peritoneal cavity and into contact with the patient's peritoneal membrane. Waste, toxins and excess water pass from the patient's bloodstream through the peritoneal membrane and into the dialysate. The transfer of waste, toxins, and excess water from the bloodstream into the dialysate occurs due to diffusion and osmosis during a dwell period as an osmotic agent in the dialysate creates an osmotic gradient across the membrane. The spent dialysate is later drained from the patient's peritoneal cavity to remove the waste, toxins and excess water from the patient.

There are various types of peritoneal dialysis therapies, including continuous ambulatory peritoneal dialysis ("CAPD") and automated peritoneal dialysis. CAPD is a manual dialysis treatment, in which the patient connects the catheter to a bag of fresh dialysate and manually infuses fresh dialysate through the catheter or other suitable access device and into the patient's peritoneal cavity. The patient disconnects the catheter from the fresh dialysate bag and allows the dialysate to dwell within the cavity to transfer waste, toxins and excess water from the patient's bloodstream to the dialysate solution. After a dwell period, the patient drains the spent dialysate and then repeats the manual dialysis procedure. Tubing sets with "Y" connectors for the solution and drain bags are available that can reduce the number of connections the patient must make. The tubing sets can include pre-attached bags including, for example, an empty bag and a bag filled with dialysate.

In CAPD, the patient performs several drain, fill, and dwell cycles during the day, for example, about four times per day. Each treatment cycle, which includes a drain, fill and dwell, takes about four hours.

Automated peritoneal dialysis is similar to continuous ambulatory peritoneal dialysis in that the dialysis treatment includes a drain, fill, and dwell cycle. However, a dialysis machine automatically performs three or more cycles of peritoneal dialysis treatment, typically overnight while the patient sleeps.

With automated peritoneal dialysis, an automated dialysis machine fluidly connects to an implanted catheter. The automated dialysis machine also fluidly connects to a source or bag of fresh dialysate and to a fluid drain. The dialysis machine pumps spent dialysate from the peritoneal cavity, through the catheter, to the drain. The dialysis machine then pumps fresh dialysate from the dialysate source, through the catheter, and into the patient's peritoneal cavity. The automated machine allows the dialysate to dwell within the cavity so that the transfer of waste, toxins and excess water from the patient's bloodstream to the dialysate solution can take place. A computer controls the automated dialysis machine so that the dialysis treatment occurs automatically when the patient is connected to the dialysis machine, for example, when the patient sleeps. That is, the dialysis system automatically and sequentially pumps fluid into the peritoneal cavity, allows for dwell, pumps fluid out of the peritoneal cavity, and repeats the procedure.

Several drain, fill, and dwell cycles will occur during the treatment. Also, a final volume "last fill" is typically used at the end of the automated dialysis treatment, which remains in the peritoneal cavity of the patient when the patient disconnects from the dialysis machine for the day. Automated peritoneal dialysis frees the patient from having to manually perform the drain, dwell, and fill steps during the day.

Conventional peritoneal dialysis solutions contain glucose as an osmotic agent to maintain the osmotic pressure of the solution higher than the physiological osmotic pressure (i.e. higher than about 285 mOsmol/kg). Glucose is a preferred osmotic agent because it provides rapid ultrafiltration rates. Other types of osmotic agents are generally known and used in addition to or as a substitute for glucose.

For example, another family of compounds capable of serving as osmotic agents in peritoneal dialysis solutions is that of glucose polymers, such as icodextrins, maltodextrins and the like. However, while these compounds are suitable for use as osmotic agents, they are also known to be sensitive to low and high pH, especially during sterilization and long-term storage.

US-6,689,393 discloses a three-part peritoneal dialysis solution. The first solution contains calcium ions, electrolyte salts and optionally glucose and is acidified to a pH of less than 4.0. The second solution contains glucose and also has a pH of less than 4.0. The third solution contains a buffer and preferably has a pH of from 8.5 to 9.0. The three solutions are mixed to give a solution having a pH of from 6.8 to 7.4.

US-2002/0012707 discloses a bicarbonate-based peritoneal dialysis solution in two parts. The first part is an alkaline bicarbonate concentrate having a pH of from 8.6 to 10.0. The second part is an acidic concentrate having a pH of from 1.0 to 3.0. The two concentrates are mixed to give a solution having a pH with an acceptable physiological range.

WO 01/89478 discloses peritoneal dialysis solutions in multiple compartment containers. The osmotic agent is stabilized with a bisulphite buffer, preferably at pH:3.2.

A need, therefore, exists for improved dialysis solutions, such as peritoneal dialysis solutions, that display greater stability, improved biocompatibility characteristics and the like.

### SUMMARY OF THE INVENTION

According to the present invention there is provided a dialysis solution according to claim 1.

The present invention relates generally to dialysis solutions and methods of making same and systems including same. More specifically, the present invention relates to peritoneal dialysis solutions that include multiple solution parts to enhance the stability, biocompatibility, and overall effectiveness of ready-to-use dialysis solutions made from an admixture of same.

For example, the present invention provides a multi-chamber bag configuration that allows the storage of chemically incompatible components, such as pH-sensitive ingredients, in a separate compartment. The pH-sensitive ingredients include glucose polymers. As previously discussed, glucose polymers can be used in addition to or as a substitute for dextrose as an osmotic agent within dialysis solutions. Applicants have recognized that the stability of the dialysis solution based with glucose polymers can be enhanced by preparing, storing and sterilizing the pH-sensitive ingredient within a separate solution part at an effective pH level for such purpose. Thus, once combined with the remaining solution parts, the overall effectiveness of the ready-to-use dialysis solution can be enhanced.

The pH-sensitive component solution part is buffered (e.g., with lactate and/or other suitable buffer), while the other two solution component parts are formulated as to obtain a bicarbonate-containing solution at physiological pH upon mixing of the respective concentrates. This can further enhance the stability of the pH-sensitive solution part. The two additional solution component parts contain an acidic concentrate that includes lactic acid, for example, and an alkaline concentrate containing a bicarbonate buffer, respectively, wherein the bicarbonate ingredient is stable without gas-barrier overpouch. The mixture of the two additional solution component parts can then be added to the pH-sensitive solution part to form the ready-to-use dialysis solution.

To this end, the present invention provides a bicarbonate containing dialysis solution. The bicarbonate containing dialysis solution includes a first part housed in a first container, the first part including an alkaline bicarbonate concentrate with a pH ranging from about 8.6 to about 10.0; a second part housed in a second container, the second part including an acidic concentrate with a pH of about 4.0 or less; and a third part housed in a third container, the third part including a buffer, a pH-sensitive ingredient including a glucose polymer and having a pH of from 4.0 to 8.6 wherein the pH-sensitive ingredient does not remain stable when sterilized in any one of the first container and the second container, and wherein the pH of the second part and the third part are effective to obtain a mixed solution with a physiologically acceptable pH, such as ranging from about 6.5 to about 7.6, when the first part, the second part and the third part are mixed together.

In an embodiment, the first container is a first chamber of a multi-chamber container, the second container is a second chamber of a multi-chamber container and the third container is a third chamber of a multi-chamber container such that the first part, the second part and the third part can be mixed within the multi-chamber container to form the mixed solution.

In an embodiment, the mixed solution comprises bicarbonate 0.5 mM to 45 mM; and calcium 0.2 mM to 2.0 mM.

In an embodiment, the mixed solution comprises bicarbonate 0.5 mM to 45 mM; calcium 0.2 mM to 2.0 mM; sodium 100 mM to 150 mM; magnesium 0 mM to 1.5 mM; potassium 0 mM to 4.5 mM; chloride 70 mM to 120 mM; lactate 0 mM to 60 mM; acetate 0 mM to 60 mM; citrate 0 mM to 60 mM; and pyruvate 0 mM to 60 mM.

In an embodiment, the bicarbonate containing dialysis solution further comprises one or more osmotic agents selected from glucose, modified starch, hydroxyethyl starch, polyols, amino acids, peptides, glycerol and the like.

In an embodiment, the first container, the second container and the third container are constructed of a gas permeable material.

In another embodiment, a three-part bicarbonate solution for peritoneal dialysis is provided. The solution includes a first part stored in a first container and including an alkaline bicarbonate concentrate with a pH ranging from about 8.6 to about 10.0; a second part stored in a second container and including an acidic concentrate with a pH less than about 4.0; and a third part stored in a third container and including a concentrate with a pH ranging from about 4.0 to about 8.6 wherein the concentrate includes a pH-sensitive osmotic agent comprising a glucose polymer and a buffer, and wherein the first part, the second part and the third part are admixed to form a mixed solution with a pH ranging from about 6.5 to about 7.6 that comprises bicarbonate 0.5 mM to 45 mM; calcium 0.2 mM to 2.0 mM; sodium 100 mM to 150 mM; magnesium 0 mM to 1.5 mM; potassium 0 mM to 4.5 mM; chloride 70 mM to 120 mM; lactate 0 mM to 60 mM; acetate 0 mM to 60 mM; citrate 0 mM to 60 mM; and pyruvate 0 mM to 60 mM.

In an embodiment, the first container is a first chamber of a multi-chamber container, the second container is a second chamber of a multi-chamber container and the third container is a third chamber of a multi-chamber container such that the first part, the second part and the third part can be mixed within the multi-chamber container to form the mixed solution.

The pH-sensitive osmotic agent includes a glucose polymer.

In an embodiment, the three-part bicarbonate dialysis solution further comprises an osmotic agent that includes a glucose, a modified starch, one or more amino acids, one or more peptides, a glycerol, the like and combinations thereof.

In an embodiment, the first container, the second container and the third container are constructed of a gas permeable material.

In an embodiment, the third part is buffered with a buffer agent that includes lactate, pyruvate, acetate, citrate, an intermediate of the KREBS cycle, the like and combinations thereof.

In an embodiment, the acidic concentrate includes lactic acid/lactate, pyruvic acid/pyruvate, acetic acid/acetate, citiric acid/citrate an intermediate of the KREBS cycle, hydrochloric acid, and combinations thereof.

In yet another embodiment, a method of preparing a dialysis solution is provided. The method includes preparing a first part housed in a first container, a second part housed in a second container and a third part housed in a third container wherein the first part includes an alkaline bicarbonate concentrate with a pH ranging from about 8.6 to about 10.0, wherein the second part includes an acidic concentrate with a pH ranging from about 4.0 or less, and wherein the third part includes a buffer and a pH-sensitive osmotic agent comprising a glucose polymer and has a pH of from 4.0 to 8.6; mixing the first part and the second part to form a solution mixture; and mixing the third part with the solution mixture to form a ready-to-use dialysis solution that has a pH ranging from about 6.5 to about 7.6.

In an embodiment, the first part, the second part, and the third part are sterilized prior to forming the ready-to-use dialysis solution.

In an embodiment, the pH-sensitive osmotic agent is separately sterilized prior to mixing with the first part and the second part.

The buffer is added to the third part to promote stabilization of the pH-sensitive osmotic agent.

In an embodiment, the buffer includes lactate, pyruvate, acetate, citrate, an intermediate of the KREBS cycle, the like and combinations thereof.

In an embodiment, the ready-to-use dialysis solution comprises bicarbonate 0.5 mM to 45 mM; calcium 0.2 mM to 2.0 mM; sodium 100 mM to 150 mM; magnesium 0 mM to 1.5 mM; potassium 0 mM to 4.5 mM; chloride 70 mM to 120 mM; lactate 0 mM to 60 mM; acetate 0 mM to 60 mM; citrate 0 mM to 60 mM; and pyruvate 0 mM to 60 mM.

In an embodiment, the ready-to-use dialysis solution further comprises an osmotic agent that includes a glucose, a modified starch, one or more amino acids, one or more peptides, a glycerol, the like and combinations thereof.

A method of providing dialysis to a patient is also disclosed. The method includes preparing a dialysis solution wherein the dialysis solution includes a first part, a second part and a third part that are admixed to form the dialysis solution. The first part includes an alkaline concentrate including bicarbonate and having a pH from about 8.6 to about 10.0; the second part includes an acidic concentrate with a pH less than about 4.0; and the third part includes an osmotic agent including a glucose polymer and has a pH ranging from about 4.0 to about 8.6 and further includes a buffer to promote stabilization of the osmotic agent. The method further includes using the dialysis solution during dialysis.

In an embodiment, the ready-to-use peritoneal dialysis solution is used during peritoneal dialysis, such as continuous ambulatory peritoneal dialysis, automated peritoneal dialysis and the like.

In yet a further embodiment, the present invention provides a system for providing dialysis. The system includes a first part housed in a first container, wherein the first part includes an alkaline bicarbonate concentrate with a pH ranging from about 8.6 to about 10.0; a second part housed in a second container, wherein the second part includes an acidic concentrate with a pH less than about 4.0; and a third part housed in a third container, wherein the third part includes a buffer and a pH-sensitive osmotic agent including a glucose polymer and having a pH ranging from about 4.0 to about 8.6. The pH-sensitive osmotic agent does not remain stable when sterilized in any one of the first container and the second container, wherein a pH of the second part and the third part are effective to obtain, when the first part, the second part and the third part are mixed together, a mixed solution with a physiologically acceptable pH, ranging from about 6.5 to about 7.6. Further, the system at least includes a tubing set that is adaptedly coupled to the first, second and third parts thereby allowing use of same during dialysis.

In still yet another embodiment, the present invention provides a three-part bicarbonate solution for peritoneal dialysis. The solution includes a first part stored in a first container and including an alkaline bicarbonate concentrate with a pH ranging from about 8.6 to about 10.0; a second part stored in a second container and including an acidic concentrate with a pH less than about 4.0; and a third part stored in a third container and including a concentrate with a pH ranging from about 4.0 to about 5.5 wherein the concentrate includes a pH-sensitive osmotic agent including a glucose polymer and a buffer to further promote stabilization of the pH-sensitive osmotic agent, and wherein the first part, the second part and the third part are capable of being admitted to form a mixed solution with a pH ranging from about 6.5 to about 7.6.

In an embodiment, the third part includes about 15 mM or less of the buffer, such as about 10 mM or less.

An advantage of the present invention is to provide improved dialysis solutions.

Another advantage of the present invention is to provide dialysis solutions with improved biocompatibility characteristics.

Yet another advantage of the present invention is to provide dialysis solutions with enhanced stability, such as during sterilization, storage and the like.

Still yet another advantage of the present invention is to provide improved dialysis solutions that include separately formulated and sterilized solution parts to promote the stabilization of a pH-sensitive component in one or more of the solution parts.

A further advantage of the present invention is to provide a lactate-buffered dialysis solution.

A still further advantage of the present invention is to provide improved methods of making dialysis solutions.

Additional features and advantages of the present invention are described in, and will be apparent from, the following Detailed Description of the Invention and the figures.

### BRIEF DESCRIPTION OF THE FIGURES

Figure 1 is a schematic representation of a multi-chambered bag for use with the dialysis solutions pursuant to an embodiment of the present invention.
Figure 2 is a schematic representation of an automated peritoneal dialysis therapy utilizing the dialysis solutions pursuant to an embodiment of the present invention.

### DETAILED DESCRIPTION OF THE INVENTION

The present invention generally relates to dialysis solutions and methods of making same and systems including same. More specifically, the present invention relates to peritoneal dialysis solutions that include at least three solution parts. The solution parts are separately formulated, sterilized and stored prior to mixing to form a ready-to-use solution. This can enhance the stability, biocompatibility, and overall effectiveness of ready-to-use solutions.

The present invention relates to bicarbonate-based dialysis solutions that are formulated from at least three separate solution parts, such as an alkaline bicarbonate concentrate in which the bicarbonate ingredient is stable without a gas barrier overpouch, an acidic concentrate containing lactic acid, for example, and a concentrate containing an osmotic agent, and/or any other solution ingredient, such as an additive, which does not remain stable when sterilized/stored in the remaining other solution compartments. The solution parts are designed to provide a final solution with an acceptable pH in the physiological range upon mixing.

The dialysis solutions of the present invention can be used in a variety of suitable applications. Preferably, the dialysis solutions are used during peritoneal dialysis, such as during continuous ambulatory peritoneal dialysis, automated peritoneal dialysis, and the like. However, it should be appreciated that the present invention can be used in a variety of different and suitable dialysis therapies to treat kidney failure. Dialysis therapy as the term or like terms are used throughout the text is meant to include and encompass any and all suitable forms of therapies that utilize the patient's blood to remove waste, toxins and excess water from the patient. Such therapies, such as hemodialysis, hemofiltration and hemodiafiltration, include both intermittent therapies and continuous therapies used for continuous renal replacement therapy (CRRT). The continuous therapies include, for example, slow continuous ultrafiltration (SCUF), continuous venovenous hemofiltration (CVVH), continuous venovenous hemodialysis (CVVHD), continuous venovenous hemodiafiltration (CVVHDF), continuous arteriovenous hemofiltration (CAVH), continuous arteriovenous hemodialysis (CAVHD), continuous arteriovenous hemodiafiltration (CAVHDF), continuous ultrafiltration periodic intermittent hemodialysis or the like. Preferably, the dialysis solutions are used during peritoneal dialysis, such as automated peritoneal dialysis, continuous ambulatory peritoneal dialysis, continuous flow peritoneal dialysis and the like. Further, although the present invention, in an embodiment, can be utilized in methods providing a dialysis therapy for patients having chronic kidney failure or disease, it should be appreciated that the present invention can be used for acute dialysis needs, for example, in an emergency room setting. Lastly, as one of skill in the art appreciates, the intermittent forms of therapy (i.e., hemofiltration, hemodialysis, peritoneal dialysis and hemodiafiltration) may be used in the in center, self/limited care as well as the home settings.

The three-part dialysis solutions of the present invention can include any suitable number, type and amount of solution components. Applicants have found that the stability of the pH-sensitive osmotic agent solution part can be enhanced by preparing, sterilizing and storing this solution part at an effective pH range and separated from the remaining other solution parts. Prior to use, the pH-sensitive solution part can be admixed with the remaining other solution parts to form the ready-to-use solution.

As defined herein, the term "pH-sensitive component" or other like terms means any suitable type of solution component that remains stable at an intermediate pH level as compared to the pH of the acidic concentrate, the bicarbonate concentrate and other like solution parts associated with a multi-part dialysis solution. For example, the pH-sensitive component can remain stable at a pH ranging from about 3.0 to about 8.6 during preparation, sterilization, storage and otherwise condition until use thereof in an embodiment. The pH-sensitive component includes an osmotic agent comprising a glucose polymer that remains stable at the pH range as discussed above. The pH can range from about 4.0 to about 5.5 according to an embodiment.

The pH-sensitive osmotic agent includes a glucose polymer. As previously discussed, glucose polymers, such as icodextrin, can be used in addition to or in place of dextrose in peritoneal dialysis solutions. In general, icodextrin is a polymer of glucose derived from the hydrolysis of corn starch. It has a molecular weight of 12-20,000 Daltons. The majority of glucose molecules in icodextrin are linearly linked with α (1-4) glucosidic bonds (>90%) while a small fraction (<10%) is linked by α (1-6) bonds.

The pH-sensitive component solution part can include any number of suitable other types of components. The pH-sensitive component part also includes a buffer, such as an organic acid including, for example, lactate, pyruvate, acetate, citrate, an intermediate of the KREBS cycle, the like and combinations thereof. The buffer addition can further enhance the stability of the pH-sensitive solution component part.

The additional solution components of the dialysis solutions of the present invention at least include a bicarbonate-based concentrate and an acidic concentrate. The bicarbonate-based concentrate can include any suitable type and amount of solution components. In an embodiment, the bicarbonate concentrate is an alkaline solution such that the bicarbonate can remain stable without the use a gas barrier overpouch or the like. The bicarbonate concentrate has a pH that ranges from about 8.6 to about 10.0, preferably about 9.0. The pH of the bicarbonate solution part can be adjusted with any suitable type of component, such as sodium hydroxide and/or the like. Illustrative examples of the bicarbonate solution part of the present invention can be found in U.S. Patent No. 6,309,673, entitled BICARBONATE-BASED SOLUTION IN TWO PARTS FOR PERITONEAL DIALYSIS OR SUBSTITUTION IN CONTINUOUS RENAL REPLACEMENT THERAPY, issued on October 30, 2001.

The acidic concentrate solution part can include any suitable type, amount and number of components. In an embodiment, the acidic concentrate includes one or more physiological acceptable acids, such as lactic acid, pyruvic acid, acetic acid, citric acid, hydrochloric acid and the like. The acidic concentrate has a pH that can be lower than the remaining other solution parts. The pH is about 4.0 or less, such as about 3.0 or less, about 2.0 or less, about 1.0 or less, and any other suitable acidic pH. In order to achieve a physiological pH upon mixing, a predetermined amount of acid (H+ ions) needs to be present in the solutions parts excluding the bicarbonate solution part for stability purposes. This can be in the form of hydrochloric acid, lactic acid, or any other physiologically acceptable acid as discussed above. The use of lactic acid, however, allows formulation of the acidic concentrate at a less acidic pH than when using concentrated hydrochloric acid. Thus, the use of an organic acid, such as lactic acid, alone or in combination with another suitable acid, such as a suitable inorganic acid including hydrochloric acid, another suitable organic acid and the like in the acidic concentrate solution can make the solution part more physiologically tolerable according to an embodiment.

The dialysis solutions of the present invention can include other solution components in addition to those components described above. For example, any one or combination of suitable solution parts can include one or more electrolytes. Examples of electrolytes include calcium, magnesium, sodium, chloride and/or the like. In an embodiment, the electrolytes are added to the pH-sensitive solution part and the acidic concentrate solution part. In another embodiment, sodium and/or chloride can be added to any one or a combination of the three solution parts including the pH-sensitive solution part, the bicarbonate-based solution part and the acidic concentrate solution part.

Examples illustrative of various embodiments of the present invention are provided below according to an embodiment of the present invention without limitation.

Three solutions were prepared according to an embodiment of the present invention. The pH of the solution parts for each of the three test solutions was measured before and after sterilization. The pH of the final mixed solutions in addition to the partial pressure of carbon dioxide (pCO₂) were also measured as described below in greater detail.

The three product configurations were produced on a small scale and sterilized under regular manufacturing conditions. The formulation of the three reconstituted solutions was the same as shown below in Table I:

**Table I**

| | g/l | mM |
|---|---|---|
| Icodextrin | 75 | - |
| Ca | - | 1.75 |
| Mg | - | 0.25 |
| Na | - | 132 |
| Cl | - | 101 |
| Bicarbonate | - | 25 |
| Lactate | - | 10 |

The difference between the three tested configurations is due from the amount of lactate present in the intermediate pH (e.g., icodextrin-containing) compartment as follows and further shown below: 0 mM (solution 1), 10 mM (solution 2), and 15 mM (solution 3). The ratio of the alkaline concentrate, the acidic concentrate, and the intermediate pH concentrate was chosen to be 1:1:2. The bicarbonate and sodium hydroxide concentrations in the alkaline bicarbonate containing concentrate were predetermined and required 26.7 mM H+ in the acidic concentrate for neutralization. Because the lactate concentration in the mixed solution was also predetermined at 10 mM, the lactate concentration in the intermediate pH compartment dictated the maximal amount of lactic acid and lactate that could be added to the acidic compartment. The 26.7 mM H+ in the acidic concentrate, required for neutralization of the alkaline concentrate, was obtained by adding the maximal amount of lactic acid, and supplementing this with hydrochloric acid, if necessary. The solution parts for each of the three test solutions are illustrated below in Table II (solution 1), Table III (solution 2) and Table IV (solution 3):

**Table II**

| **Comparative Solution 1** | **Volume** | **g/l** | **mM** | **pH before sterilization** | **pH after sterilization** |
|---|---|---|---|---|---|
| NaCl | 500 ml | 7.25 | 124 | 9.10 | 9.10 |
| NaHCO₃ | | 9.29 | 111 | | |
| NaOH (adj) | | - | 16.1 | | |
| Lactic Acid | 500 ml | 2.41 | 26.7 | 3.51 | 3.47 |
| Na Lactate | | 1.49 | 13.3 | | |
| Icodextrin | 1000 ml | 150 | - | 4.79 | 4.31 |
| CaCl₂.2H₂0 | | 0.52 | 3.5 | | |
| MgCl₂.6H₂0 | | 0.10 | 0.5 | | |
| NaCl | | 7.71 | 132 | | |
| | | | | | |

| | pH (RT) | pH 25°C | pH 37°C | pCO₂ 25°C | pCO₂ 37°C |
|---|---|---|---|---|---|
| **Mixed Solution 1** | 7.26 | 7.36 | 7.20 | 35.7 mm Hg | 63.8 mm Hg |

**Table III**

| **Solution 2** | **Volume** | **g/l** | **mM** | **pH before sterilization** | **pH after sterilization** |
|---|---|---|---|---|---|
| NaCl | 500 ml | 6.90 | 118 | 9.10 | 9.09 |
| NaHCO₃ | | 9.29 | 111 | | |
| NaOH (adj) | | - | 16.1 | | |
| Lactic Acid | 500 ml | 1.80 | 20.0 | 2.17 | 2.16 |
| HCl | | 0.25 | 6.7 | | |
| Icodextrin | 1000 ml | 150 | - | 5.12 | 4.97 |
| CaCl₂.2H₂0 | | 0.52 | 3.5 | | |
| MgCl₂.6H₂0 | | 0.10 | 0.5 | | |
| Na Lactate | | 1.12 | 10 | | |
| NaCl | | 7.71 | 132 | | |
| | | | | | |

| | pH (RT) | pH 25°C | pH 37°C | pCO₂ 25°C | pCO₂ 37°C |
|---|---|---|---|---|---|
| **Mixed Solution 2** | 7.25 | 7.30 | 7.15 | 40.4 mm Hg | 72.2 mm Hg |

**Table IV**

| **Solution 3** | **Volume** | **g/l** | **mM** | **pH before sterilization** | **pH after sterilization** |
|---|---|---|---|---|---|
| NaCl | 500 ml | 6.31 | 108 | 9.11 | 9.09 |
| NaHCO₃ | | 9.29 | 111 | | |
| NaOH (adj) | | - | 16.1 | | |
| Lactic Acid | 500 ml | 0.90 | 10.0 | 1.83 | 1.76 |
| HCl | | 0.61 | 16.7 | | |
| Icodextrin | 1000 ml | 150 | - | 5.13 | 4.99 |
| CaCl₂.2H₂0 | | 0.52 | 3.5 | | |
| MgCl₂.6H₂0 | | 0.10 | 0.5 | | |
| NaLactate | | 1.68 | 15 | | |
| NaCl | | 7.71 | 132 | | |
| | | | | | |

| | pH (RT) | pH 25°C | pH 37°C | pCO₂ 25°C | pCO₂ 37°C |
|---|---|---|---|---|---|
| **Mixed Solution 3** | 7.21 | 7.27 | 7.11 | 43.4 mm Hg | 77.6 mm Hg |

As shown in Tables II-IV, the pH results obtained on non-sterilized and sterilized solutions demonstrated that the addition of small amounts of lactate to the intermediate pH (e.g., icodextrin-containing) compartment prevents a pH-drop during sterilization. The results also show that the pH of the three acidic concentrates varies in function of the composition of lactic acid, lactate and hydrochloric acid. The mixed solutions displayed both a physiological pH and a physiological pCO₂ as further illustrated above.

It should be appreciated that the solution parts of the dialysis solutions of the present invention can be housed or contained in any suitable manner such that the dialysis solutions can be effectively prepared, sterilized, stored and used. In an embodiment, the present invention includes a multi-part dialysis solution in which three or more solution parts are formulated, stored and sterilized separately, and then mixed just prior to use. A variety of containers can be used to house the various parts of the dialysis solution, such as separate containers (e.g., flasks or bags) that are connected by a suitable fluid communication mechanism. In an embodiment, a multi-chamber container or bag can be used to house the separate solution parts including the pH-sensitive solution part, the bicarbonate-based solution part and the acidic concentrate solution part. In an embodiment, the separate components are mixed within the multi-chamber bag prior to use, such as during continuous ambulatory peritoneal dialysis.

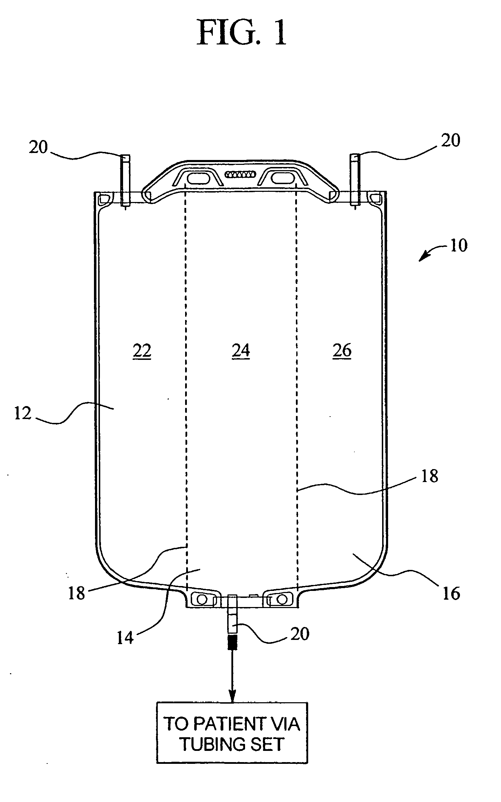

Figure 1 illustrates a suitable container for storing, formulating, mixing and administering a dialysis solution, such as during continuous ambulatory peritoneal dialysis, according to an embodiment of the present invention. The multi-chamber bag 10 has a first chamber 12, a second chamber 14, and a third chamber 16. The interior of the container is divided by a peelable seal 18 into the three chambers. The peelable seal allows for the mixing of the solution components that are contained within each chamber as described in greater detail below. It should be appreciated that any suitable device, on its own or in combination with the peelable seal, can be used to allow mixing of the solution components. For example, the bag 10 can be divided into separate chambers by a heat seal material where connectors are provided to allow mixing of the solution components between the chambers. In an embodiment, the connector includes a frangible connector, any suitable other type of connector and combinations thereof. It should be further appreciated that the volume ratio of the chambers can be arranged and configured in any suitable manner. As further shown in Fig. 1, the bag 10 includes a port 20 attached to each chamber through which a respective solution component or mixture thereof can flow.

The multi-chamber bag 10 contains the three solution parts as previously discussed. In an embodiment, the alkaline bicarbonate solution part 22 is contained within the first chamber 12; the acidic concentrate solution part 24 is contained within the second chamber 14; and the pH-sensitive or intermediate pH solution part 26 is contained within the third chamber 16. The bicarbonate-based solution part 22 is then mixed with the acidic concentrate solution part 24. This mixture is further mixed with the pH-sensitive solution part to form a ready-to-use dialysis solution within the multi-chambered bag 10. The ready-to-use solution can then be administered to a patient through a respective port in any suitable manner.

It should be appreciated that the multi-chamber bag can be modified and configured in any suitable way to separately contain the solution parts and further allow for admixing of same. Further, the solution parts can be admixed in any suitable sequence. An example of a multi-chamber bag can be found in U.S. Patent No. 4,465,488, entitled COLLAPSIBLE MULTI-CHAMBER MEDICAL FLUID CONTAINER, issued on August 14, 1984; and U.S. Patent No. 4,396,383, entitled MULTIPLE CHAMBER SOLUTION CONTAINER INCLUDING POSITIVE TEST FOR HOMOGENOUS MIXTURE, issued on August 2, 1983. In an embodiment, the multi-chamber bag can be made of a gas permeable material, such as polypropylene, polyvinyl chloride and the like.

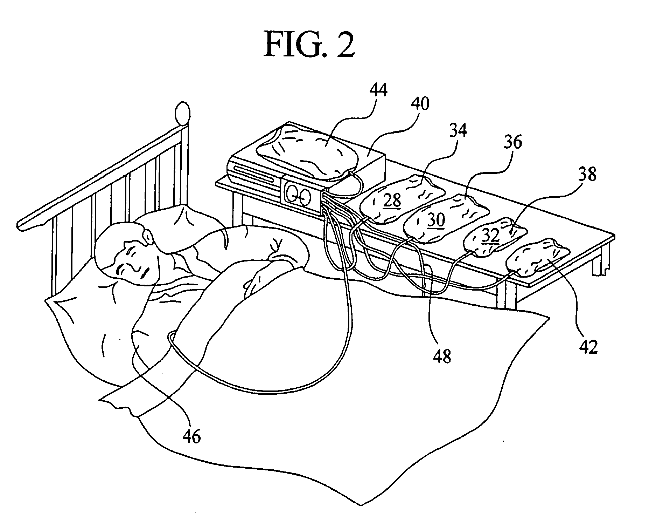

In another embodiment, the solution parts can be prepared, sterilized and stored in separate containers and then mixed via an admix device prior to use, such as applied during automated peritoneal dialysis. As shown in Figure 2, the bicarbonate concentrate 28, the acidic concentrate 30, and the pH-sensitive or intermediate pH concentrate 32 are stored in respective separate containers 34, 36 and 38 or bags which are fluidly connected to an admix device 40 suitable for use during automated peritoneal dialysis, an example of which includes ADMIX HOMECHOICE by BAXTER INTERNATIONAL, INC.. In addition to the solution concentrates, other solution bags, such as solution bag 42, can also be used during dialysis therapy as generally known. In an embodiment, an effective amount of the solution concentrates are drawn from each respective container and into a heater bag 44 where the solution concentrates can be mixed and heated prior to infusion into a patient 46 during dialysis therapy. As further shown in Figure 2, a drain line 48 is coupled to the admix device 40 from which waste fluids can be removed from the patient during therapy.

In an embodiment, the present invention provides systems for providing dialysis. The systems include multi-part solution components, such as the first, second and third solutions parts, that can be admixed to form a ready-to-use solution for dialysis as previously discussed. Further, the systems at least include a tubing set or the like adaptedly coupled to the various solution parts thereby allowing use of same during dialysis, such as continuous peritoneal dialysis and automated peritoneal dialysis as discussed above. The tubing set can be configured in any suitable manner and be made of any suitable material or materials. As shown in Fig. 1, the ready-to-use solution that is prepared in the multi-chamber bag 10 can be sent to a patient via a tubing set 50 during continuous ambulatory peritoneal dialysis. In Fig. 2, the solution parts are sent via tubing to a mixing device and subsequently to the patent during automated peritoneal dialysis.

It should be appreciated that the present invention can be modified in any suitable manner. For example, various osmotic agents or additives can be added to any one or a combination of the solution parts. In an embodiment, glucose can be added to the acidic concentrate and, optionally, amino acids can be added to the alkaline concentrate. Glucose can then be sterilized at an effectively low pH, thus preventing the formation of glucose degradation products. The amino acids can be sterilized separately in the alkaline solution part.

As previously discussed, the pH sensitive osmotic agent can be sterilized in its own compartment at an appropriately buffered pH. Alternatively the pH sensitive component (e.g., the pH-sensitive osmotic agent) can be a component which is chemically incompatible with components present in other solution concentrates and thus has to be sterilized in a separate compartment. As previously discussed, the multi-chamber bag can be configured in any suitable manner. For example, the volume ratios of the different and separate chambers can vary depending on the application thereof.

It should be understood that various changes and modifications to the presently preferred embodiments described herein will be apparent to those skilled in the art.

## Claims

1. A dialysis solution comprising:
a first part including a bicarbonate concentrate having a pH of from 8.6 to 10.0;
a second part housed separately from the first part, the second part including an acidic concentrate having a pH of 4.0 or less; and
a third part housed separately from the first part and the second part, the third part including a buffer and an osmotic agent comprising a glucose polymer, the third part having a pH of from 4.0 to 8.6.

2. The dialysis solution of claim 1, wherein the bicarbonate concentrate does not require a gas barrier over-pouch prior to use.

3. The dialysis solution of claim 1 or 2, wherein the solution as mixed comprises:
bicarbonate 0.5 mM to 45mM; and
calcium 0.2 mM to 2.0 mM.

4. The dialysis solution of claim 3, wherein the solution as mixed comprises:
bicarbonate 0.5 mM to 45mM;
calcium 0.2 mM to 2.0 mM;
sodium 100 mM to 150 mM;
magnesium 0 mM to 1.5 mM;
potassium 0 mM to 4.5 mM;
chloride 70 mM to 120 mM;
lactate 0 mM to 60 mM;
acetate 0 mM to 60 mM;
citrate 0 mM to 60 mM; and
pyruvate 0 mM to 60 mM.

5. The dialysis solution of any one of claims 1 to 4, wherein the buffer is selected from the group consisting of lactate, pyruvate, acetate, citrate, an intermediate of the KREBS cycle, and combinations thereof.

6. The dialysis solution of any one of the preceding claims, wherein the third part includes 15mM or less of the buffer.

7. The dialysis solution of any one of the preceding claims, wherein the acidic concentrate is selected from the group consisting of lactic acid/lactate, pyruvic acid/pyruvate, acetic acid/acetate, citric acid/citrate, an intermediate of the KREBS cycle, hydrochloric acid, and combinations thereof.

8. The dialysis solution of any one of the preceding claims, wherein the first, second and third parts are housed in first, second and third chambers of a multi-chamber container and wherein the first, second and third parts can be mixed within the multi-chamber container.

9. The dialysis solution of any one of the preceding claims, wherein the glucose polymer is icodextrin.

10. The dialysis solution of any one of the preceding claims, wherein the pH of the second part and the pH of the third part are effective to obtain, when the first part, the second part and the third part are mixed together, a mixed solution with a physiologically acceptable pH that ranges from 6.5 to 7.6.

## Patentansprüche

1. Dialyselösung, umfassend:
einen ersten Teil, welcher ein Hydrogencarbonatkonzentrat mit einem pH von 8,6 bis 10,0 einschließt,
einen zweiten Teil, welcher entfernt von dem ersten Teil untergebracht ist, wobei der zweite Teil ein saures Konzentrat mit einem pH von 4,0 oder weniger einschließt, und
einen dritten Teil, welcher getrennt von dem ersten Teil und dem zweiten Teil untergebracht ist, wobei der dritte Teil einen Puffer und ein Osmosemittel, umfassend ein Glucosepolymer, einschließt, wobei der dritte Teil einen pH von 4,0 bis 8,6 aufweist.

2. Dialyselösung nach Anspruch 1, wobei das Hydrogencarbonatkonzentrat keinen Gasbarrieren-Umverpackung vor der Verwendung erfordert.

3. Dialyselösung nach Anspruch 1 oder 2, wobei die Lösung, wie gemischt, umfaßt:
Hydrogencarbonat 0,5 mM bis 45 mM, und
Calcium 0,2 mM bis 2,0 mM.

4. Dialyselösung nach Anspruch 3, wobei die Lösung, wie gemischt, umfaßt:
Hydrogencarbonat 0,5 mM bis 45 mM,
Calcium 0,2 mM bis 2,0 mM,
Natrium 100 mM bis 150 mM,
Magnesium 0 mM bis 1,5 mM,
Kalium 0 mM bis 4,5 mM,
Chlorid 70 mM bis 120 mM,
Lactat 0 mM bis 60 mM,
Acetat 0 mM bis 60 mM,
Citrat 0 mM bis 60 mM, und
Pyruvat 0 mM bis 60 mM.

5. Dialyselösung nach einem der Ansprüche 1 bis 4, wobei der Puffer ausgewählt ist aus der Gruppe, bestehend aus Lactat, Pyruvat, Acetat, Citrat, einem Intermediat des KREBS-Zyklus, und Kombinationen davon.

6. Dialyselösung nach einem der vorhergehenden Ansprüche, wobei der dritte Teil 15 mM oder weniger des Puffers einschließt.

7. Dialyselösung nach einem der vorhergehenden Ansprüche, wobei das saure Konzentrat ausgewählt ist aus der Gruppe, bestehend aus Milchsäure/Lactat, Brenztraubensäure/Pyruvat, Essigsäure/Acetat, Zitronensäure/Citrat, einem Intermediat des KREBS-Zyklus, Chlorwasserstoffsäure, und Kombinationen davon.

8. Dialyselösung nach einem der vorhergehenden Ansprüche, wobei der erste, zweite und dritte Teil in einer ersten, zweiten und dritten Kammer eines Mehrkammerbehälters untergebracht sind, und wobei der erste, zweite und dritte Teil innerhalb des Mehrfachkammerbehälters gemischt werden können.

9. Dialyselösung nach einem der vorhergehenden Ansprüche, wobei das Glukosepolymer Icodextrin ist.

10. Dialyselösung nach einem der vorhergehenden Ansprüche, wobei der pH des zweiten Teils und der pH des dritten Teils wirksam sind, wenn der erste Teil, der zweite Teil und der dritte Teil zusammengemischt werden, eine gemischte Lösung mit einem physiologisch verträglichen pH zu erhalten, welcher von 6,5 bis 7,6 reicht.

## Revendications

1. Solution de dialyse comprenant :
un premier composant comportant un concentré de bicarbonate présentant un pH compris entre 8,6 et 10,0 ;
un deuxième composant contenu séparément par rapport au premier composant, le deuxième composant comportant un concentré acide présentant un pH inférieur ou égal à 4,0 ; et
un troisième composant contenu séparément par rapport au premier composant et au deuxième composant, le troisième composant comportant un tampon et un agent osmotique comprenant un polymère de glucose, le troisième composant présentant un pH compris entre 4,0 et 8,6.

2. Solution de dialyse selon la revendication 1, dans laquelle le concentré de bicarbonate ne nécessite pas de sur-poche de barrière au gaz, avant utilisation.

3. Solution de dialyse selon la revendication 1 ou 2, dans laquelle la solution après mélange comprend :
de 0,5 mM à 45 mM de bicarbonate ; et
de 0,2 mM à 2,0 mM de calcium.

4. Solution de dialyse selon la revendication 3, dans laquelle la solution après mélange comprend :
de 0,5 mM à 45 mM de bicarbonate ;
de 0,2 mM à 2,0 mM de calcium ;
de 100 mM à 150 mM de sodium ;
de 0 mM à 1,5 mM de magnésium ;
de 0 mM à 4,5 mM de potassium ;
de 70 mM à 120 mM de chlorure ;
de 0 mM à 60 mM de lactate ;
de 0 mM à 60 mM d'acétate ;
de 0 mM à 60 mM de citrate ; et
de 0 mM à 60 mM de pyruvate.

5. Solution de dialyse selon l'une quelconque des revendications 1 à 4, dans laquelle le tampon est sélectionné à partir du groupe constitué par du lactate, du pyruvate, de l'acétate, du citrate, un produit intermédiaire du cycle de Krebs, et des mélanges de ceux-ci.

6. Solution de dialyse selon l'une quelconque des revendications précédentes, dans laquelle le troisième composant comporte 15 mM ou moins du tampon.

7. Solution de dialyse selon l'une quelconque des revendications précédentes, dans laquelle le concentré acide est sélectionné à partir du groupe constitué par l'acide lactique/lactate, l'acide pyruvique/pyruvate, l'acide acétique/acétate, l'acide citrique/citrate, un produit intermédiaire du cycle de Krebs, l'acide chlorhydrique, et des mélanges de ceux-ci.

8. Solution de dialyse selon l'une quelconque des revendications précédentes, dans laquelle les premier, deuxième et troisième composants sont contenus dans des première, deuxième et troisième chambres d'un conteneur à plusieurs chambres et dans laquelle les premier, deuxième et troisième composants peuvent être mélangés à l'intérieur du conteneur à plusieurs chambres.

9. Solution de dialyse selon l'une quelconque des revendications précédentes, dans laquelle le polymère de glucose est de l'icodextrine.

10. Solution de dialyse selon l'une quelconque des revendications précédentes, dans laquelle le pH du deuxième composant et le pH du troisième composant permettent d'obtenir, lorsque le premier composant, le deuxième composant et le troisième composant sont mélangés ensemble, une solution mélangée présentant un pH physiologiquement compatible qui est compris entre 6,5 et 7,6.
